# EUROPÄISCHE PATENTSCHRIFT

(11) **EP 0 865 441 B1**
(45) Veröffentlichungstag und Bekanntmachung des Hinweises auf die Patenterteilung: **14.02.2001**
(21) Anmeldenummer: 96939921.1
(22) Anmeldetag: 25.11.1996
(51) Int. Cl.: C07D 417/12, C07D 285/06, A01N 43/828

(54) **1,2,3-THIADIAZOLCARBONSÄURE(THIO)ESTER UND IHRE VERWENDUNG ALS SCHÄDLINGSBEKÄMPFUNGSMITTEL ODER ALS MIKROBIZIDE**
1,2,3-THIADIAZOLE CARBOXYLIC ACID (THIO)ESTERS AND THE USE THEREOF AS A PEST CONTROL AGENT OR AS A MICROBICIDE
(THIO)ESTERS D'ACIDE 1,2,3-THIADIAZOLCARBOXYLIQUE ET LEUR UTILISATION SOUS FORME DE PESTICIDES OU DE MICROBICIDES

(30) Priorität: 07.12.1995 DE 19545637
(43) Veröffentlichungstag der Anmeldung: 23.09.1998
(73) Patentinhaber: BAYER AG, 51368 Leverkusen (DE)
(72) Erfinder: ALIG, Bernd, D-53639 Königswinter (DE); HÄNSSLER, Gerd, D-51381 Leverkusen (DE); TURBERG, Andreas, D-40699 Erkrath (DE); LONDERSHAUSEN, Michael, D-40699 Erkrath (DE)
(86) Internationale Anmeldenummer: EP9605198
(87) Internationale Veröffentlichungsnummer: WO9720840

(56) Entgegenhaltungen:
- EP-A- 0 098 486
- WO-A-95/29161
- WO-A-96/29871
- US-A- 3 776 907
- US-A- 4 341 551
- US-A- 4 829 072
- PESTICIDE SCIENCE, Bd. 43, Nr. 4, April 1995, BARKING GB, Seiten 358-61, XP000535748 G. HÖFLE ET AL.: "The synthesis and fungicidal activity of derivatives of soraphen A substituted at position 12"

## Beschreibung

Die vorliegende Erfindung betrifft neue 1,2,3-Thiadiazolcarbonsäure(thio)ester, ein Verfahren zu deren Herstellung und deren Verwendung zum Schutz von Pflanzen gegen Befall durch unerwünschte Mikroorganismen und tierische Schädlinge.

Es sind bereits bestimmte 1,2,3-Thiadiazol-5-carbonsäure-Derivate mit herbizider und pflanzenwuchsregulierender Wirkung bekannt geworden (vgl. DE-A 27 28 523). Ein Einsatz derartiger Stoffe gegen unerwünschte Mikroorganismen und tierische Schädlinge im Pflanzenschutz wurde aber bisher noch nicht beschrieben.

Weiterhin sind aus der EP - A 0 098 486, der US - A 4 829 072,der US - A 3 776 907, der US - A 4 341 551 und aus Pesticide Science 43, 358 - 361 ( 1995 ) zahlreiche fungizid bzw. pharmakologisch wirksame 1,2,3 - Thiadiazolcarbonsäure ( thio ) ester bekannt, in denen die Säuregruppe direkt mit einem Heterocyclus verbunden ist. Entsprechende Stoffe, die zwischen der Säuregruppe und dem Heterocyclus noch ein Brückenglied enthalten, werden aber nicht offenbart.

Ferner ist aus der vorgängigen, aber nicht vorveröffentlichten Patentanmeldung gemäß WO 96 - 29 871 eine Gruppe von 1,2,3 - Thiadiazolcarbonsäure - Derivaten mit fungiziden Eigenschaften bekannt. Im einzelnen genannt werden Verbindungen, in denen ein Heterocyclus der Formel 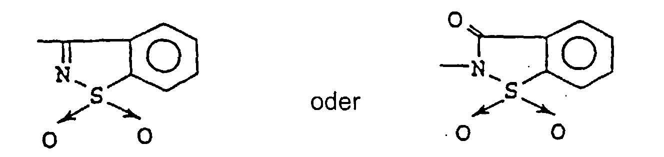 über eine -CH₂--CH₂- oder - CH₂--CH₂-O- Brücke mit der Carboxylgruppe verknüpft ist. Andere Stoffe dieses speziellen Typs werden aber nicht erwähnt.

Schließlich betrifft die Parallelanmeldung gemäß WO 97 - 20 465 auch 1,2,3 - Thiadiazolcarbonsäureester, deren Estereinheit aus einem bestimmten Carbocyclus besteht, der mit einem Heterocyclus annelliert ist. Entsprechende Stoffe, die einen direkt oder über ein Brückenglied gebundenen Heterocyclus aufweisen, werden aber nicht beschrieben.

Es wurden nun neue 1,2,3-Thiadiazolcarbonsäure(thio)ester der Formel 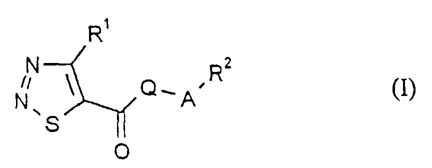 in welcher
- A: für Methylen, Ethan-1,1-diyl, Ethan-1,2-diyl, Propan-1,3-diyl, Butan-1,3-diyl, Ethen-1,2-diyl, Propen-1,3-diyl, Phenylmethan-1,1-diyl, 1-(2,4-dichloro-phenyl)-ethan-1,2-diyl steht,
oder für 1 - Phenyl-propan-1,3-diyl steht, wobei der Rest R² an die 3-Position gebunden ist,
oder für 1-( 4-Fluorphenyl ) - propan -1,3- diyl steht, wobei der Rest R² an die 3-Position gebunden ist,
oder für die Gruppen
-CH₂-O-CH₂-, -CH₂-S-CH₂-, 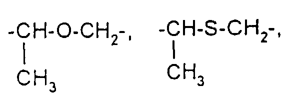 -CH=CH-CH₂-O-CH₂- und -CH=CH-CH₂-S-CH₂-, sowie für die Gruppen -CH₂-O-, -CH₂-CH₂-O-, -CH₂-S- und -CH₂-CH₂-S-
steht, wobei das Heteroatom jeweils mit dem Rest R² verbunden ist,
- Q: für Sauerstoff oder Schwefel steht,
- R¹: für Wasserstoff, Methyl, Ethyl, n-Propyl, Isopropyl, n-Butyl, Trichlormethyl, Trifluormethyl, Fluormethyl, Difluormethyl oder für Phenyl oder Naphthyl, wobei jeder dieser beiden zuletzt genannten Reste einfach bis dreifach, gleichartig oder verschieden substituiert sein kann durch Fluor, Chlor, Brom, Iod, Cyano, Nitro, Methyl, Ethyl, Methoxy, Ethoxy, Trichlormethyl, Trifluormethyl und/oder Trifluormethoxy steht und
- R²: für Pyrrolyl, Furyl, Thienyl, Imidazolyl, Pyrazolyl, Triazolyl, Oxazolyl, Isoxazolyl, Thiazolyl, Isothiazolyl, Oxadiazolyl, Thiadiazolyl, Tetrazolyl, Pyridyl, Pyrimidyl, Pyridazinyl, Pyrazinyl, Triazinyl, Indolyl, Benzimidazolyl, Benzothiazolyl, Benzotriazolyl oder Benzodioxanyl, Oxetanyl, Tetrahydrofuryl, Perhydropyranyl, Pyrrolidinyl, Piperidinyl, Morpholinyl, Dioxolanyl, Dioxanyl, Hexahydroazepinyl, Chinuclidinyl, Benzofuranyl, Benzothienyl,
Oxiranyl, Thiazinyl, Dihydrothiazinyl oder Tetrahydrothiazinyl steht, wobei diese Reste einfach bis dreifach, gleichartig oder verschieden substituiert sein können durch Fluor, Chlor, Brom, Iod, Cyano, Nitro, Amino, Hydroxy, Formyl, Carboxy, Carbamoyl, Thiocarbamoyl, Methyl, Ethyl, n-oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl, Ethoximinoethyl, Phenyl, Chlorphenyl, Methylphenyl, Phenoxy, Chlorphenoxy, Fluorphenoxy, Methylphenoxy, Cyclopropyl, Cyclobutyl, Cyclopentyl, Cyclohexyl, Dioxolanyl, das seinerseits einfach oder zweifach durch Methyl substituiert sein kann, und/oder durch Dioxanyl, das seinerseits einfach oder zweifach durch Methyl substituiert sein kann,
und/oder auch in der Weise substituiert sein können, daß jeweils zwei benachbarte Substituenten einen ankondensierten, carbocyclischen Ring mit fünf oder sechs Ringgliedern bilden, wobei der Carbocyclus seinerseits einfach bis vierfach durch Fluor, Chlor, Brom und/oder Methyl substituiert sein kann,
und/oder auch in der Weise substituiert sein können, daß jeweils zwei benachbarte Substituenten einen ankondensierten heterocyclischen Ring mit fünf oder sechs Ringgliedern und 1 oder 2 Sauerstoff-, Schwefel- und/oder Stickstoffatomen bilden, wobei der Heterocyclus seinerseits einfach oder zweifach durch Fluor, Chlor, Brom und/oder Methyl substituiert sein kann,
sowie deren Säureadditions-Salze und Metallsalz-Komplexe gefunden.

Weiterhin wurde gefunden, daß man 1,2,3-Thiadiazolcarbonsäure(thio)ester der Formel (I) sowie deren Säureadditions-Salze und Metallsalz-Komplexe erhält, wenn man 1,2,3-Thiadiazolcarbonsäure-halogenide der Formel 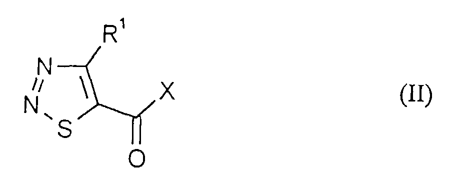 in welcher
- R¹: die oben angegebene Bedeutung hat und
- X: für Halogen steht,
mit (Thio)Alkoholen der Formel

H-Q-A-R² (III)

in welcher
- A, Q und R²: die oben angegebenen Bedeutungen haben,
gegebenenfalls in Gegenwart eines Säurebindemittels und gegebenenfalls in Gegenwart eines Verdünnungsmittels, umsetzt, und gegebenenfalls anschließend an die so erhaltenen Verbindungen der Formel (I) eine Säure oder ein Metallsalz addiert.

Schließlich wurde gefunden, daß die 1,2,3-Thiadiazolcarbonsäure(thio)ester der Formel (I) sowie deren Säureadditions-Salze und Metallsalz-Komplexe sehr gut zum Schutz von Pflanzen gegen Befall durch unerwünschte Mikroorganismen und tierische Schädlinge verwendbar sind. Die erfindungsgemäßen Stoffe eignen sich sowohl zur Erzeugung von Resistenz in Pflanzen gegen Befall durch unerwünschte Mikroorganismen als auch als Mikrobizide zur direkten Bekämpfung der Mikroorganismen.

Überraschenderweise eignen sich die erfindungsgemäßen Stoffe besser zur Bekämpfung tierischer Schädlinge und zum Schutz von Pflanzen gegen Befall durch unerwünschte Mikroorganismen als die konstitutionell ähnlichsten, vorbekannten Substanzen gleicher Wirkungsrichtung.

Die erfindungsgemäßen Stoffe können gegebenenfalls als Mischungen von verschiedenen möglichen isomeren Formen, z.B. in Form von Stereoisomeren, wie E- und Z-Isomeren oder auch optischen Isomeren, vorliegen. Die Erfindung betrifft sowohl E- und Z-Isomere als auch optische Isomere, und ebenso beliebige Mischungen dieser Isomeren.

Erfindungsgemäße Stoffe sind auch Additionsprodukte aus Säuren und denjenigen 1,2,3-Thiadiazolcarbonsäure(thio)estern der Formel (I), in denen A, Q, R¹ und R² diejenigen Bedeutungen haben, die für diese Reste genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Schwefelsäure, Salpetersäure, mono- und bifunktionelle Carbonsäuren und Hydroxycarbonsäuren, wie z.B. Essigsäure, Maleinsäure, Bernsteinsäure, Fumarsäure, Weinsäure, Zitronensäure, Salicylsäure, Sorbinsäure und Milchsäure, sowie Sulfonsäuren, wie z.B. p-Toluolsulfonsäure, 1,5-Naphthalindisulfonsäure, außerdem Saccharin und Thiosaccharin.

Erfindungsgemäße Verbindungen sind auch Additionsprodukte aus Salzen von Metallen der II. bis IV. Haupt- und der I. und II. sowie IV. bis VIII. Nebengruppe des Periodensystems der Elemente und denjenigen 1,2,3-Thiadiazolcarbonsäure(thio)estern der Formel (I), in denen A, Q, R¹ und R² diejenigen Bedeutungen haben, die für diese Reste genannt wurden.

Hierbei sind Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Als Beispiele für erfindungsgemäße Stoffe seien die in den folgenden Tabellen aufgeführten 1,2,3-Thiadiazolcarbonsäure(thio)ester genannt.

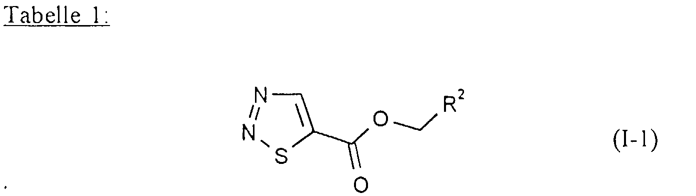

wobei R² für die folgenden Substituenten steht:

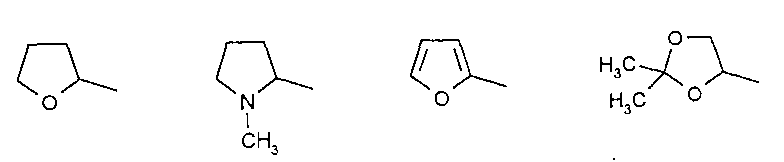

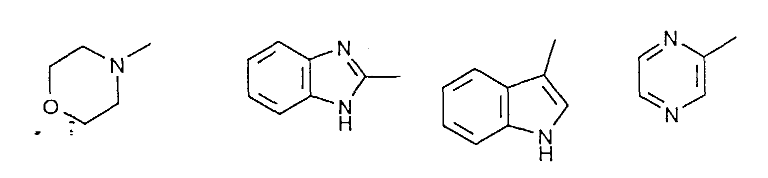

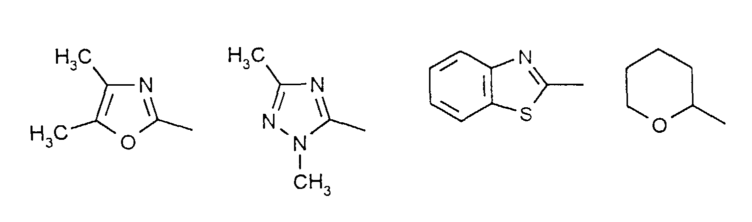

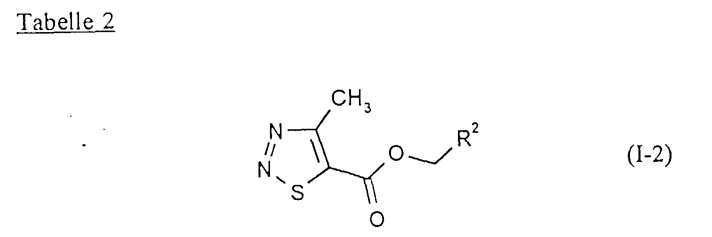

wobei R² für die in Tabelle 1 genannten Substituenten steht.

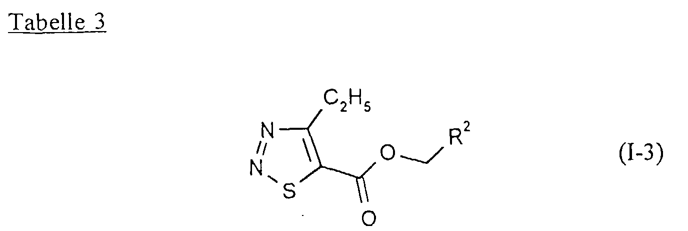

wobei R² für die in Tabelle 1 genannten Substituenten steht.

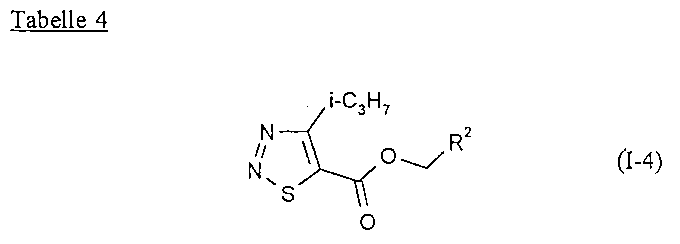

wobei R² für die in Tabelle 1 genannten Substituenten steht.

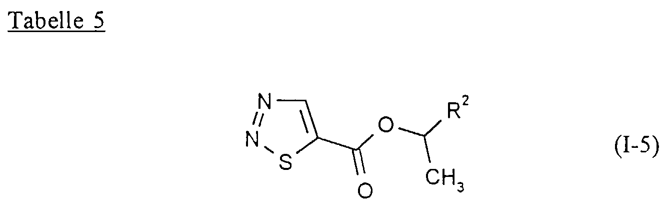

wobei R² für die in Tabelle 1 genannten Substituenten steht.

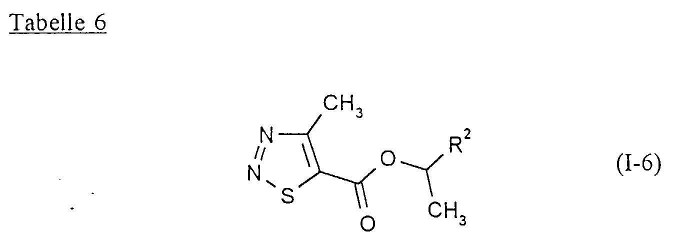

wobei R² für die in Tabelle 1 genannten Substituenten steht.

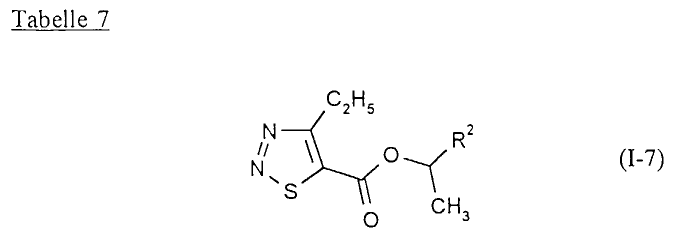

wobei R² für die in Tabelle 1 genannten Substituenten steht.

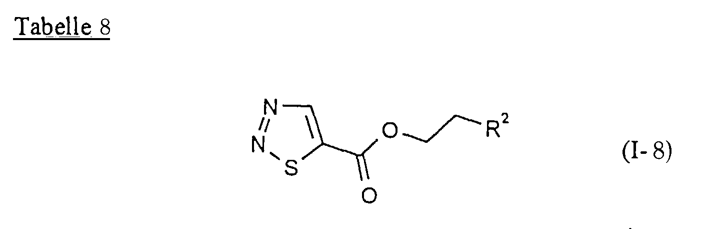

wobei R² für die in Tabelle 1 genannten Substituenten steht.

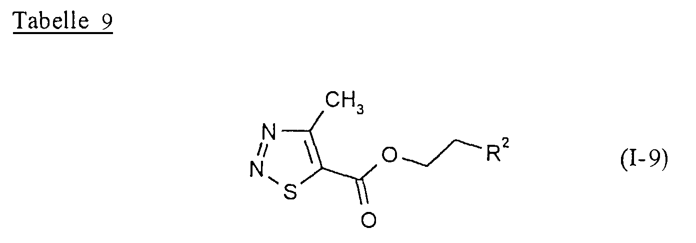

wobei R² für die in Tabelle 1 genannten Substituenten steht.

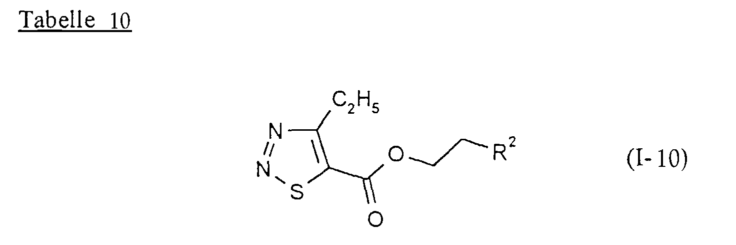

wobei R² für die in Tabelle 1 genannten Substituenten steht.

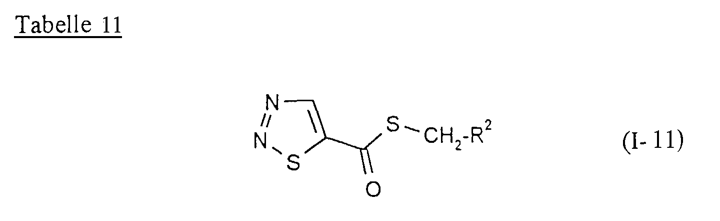

wobei R² für die folgenden Substituenten steht:

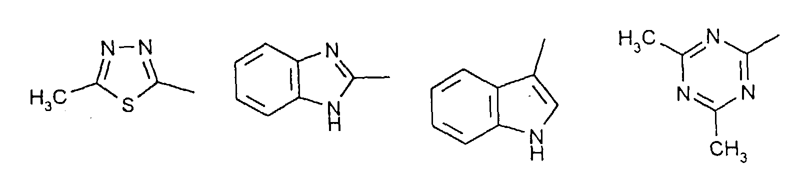

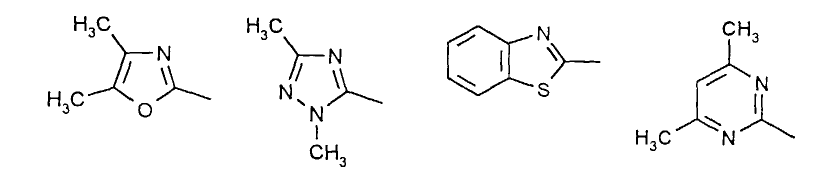

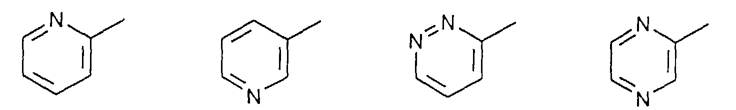

Verwendet man 4-Methyl-1,2,3-thiadiazolcarbonsäurechlorid und Furfurylalkohol als Ausgangsstoffe, so läßt sich der Verlauf des erfindungsgemäßen Verfahrens durch das folgende Formelschema veranschaulichen:

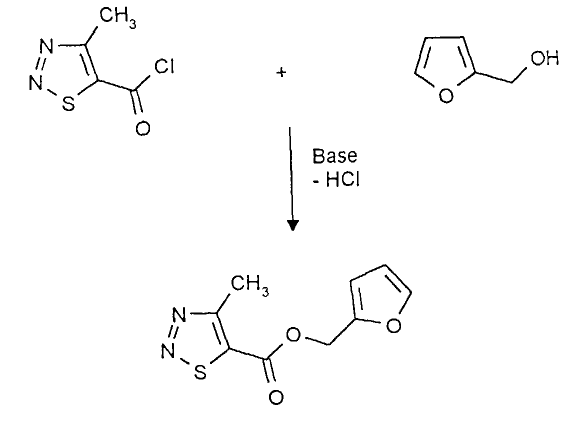

Die zur Durchführung des erfindungsgemäßen Verfahrens als Ausgangsstoffe benötigten 1,2,3-Thiadiazolcarbonsäure-halogenide sind durch die Formel (II) allgemein definiert. In dieser Formel hat R¹ diejenigen Bedeutungen, die bereits im Zusammenhang mit der Beschreibung der erfindungsgemäßen Verbindungen der Formel (I) für R¹ angegeben wurden. X steht vorzugsweise für Chlor.

Die Ausgangsstoffe der Formel (II) sind bekannt oder können nach bekannten Verfahren hergestellt werden (vgl. z. B. DE-A 2728523 oder J. Heterocyclic Chem. 1976, 301).

Die zur Durchführung des erfindungsgemäßen Verfahrens weiterhin als Ausgangsstoffe benötigten (Thio)Alkohole sind durch die Formel (III) allgemein definiert. In dieser Formel haben A, Q und R² diejenigen Bedeutungen, die bereits im Zusammenhang mit der Beschreibung der erfindungsgemäßen Verbindungen der Formel (I) für A, Q und R² angegeben wurden.

Die (Thio)Alkohole der Formel (III) sind bekannte Reagenzien der organischen Chemie.

Als Säurebindemittel kommen bei der Durchführung des erfindungsgemäßen Verfahrens alle üblichen anorganischen und organischen Basen in Frage. Vorzugsweise verwendbar sind Erdalkali- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natriumethylat, Kalium-tert.-butylat, Natriumhydroxid, Kaliumhydroxid, Natriumacetat, Kaliumacetat, Calciumacetat, Natriumcarbonat, Kaliumcarbonat, Kaliumhydrogencarbonat, Natriumhydrogencarbonat, ferner Ammonium-Verbindungen, wie Ammoniumhydroxid, Ammoniumacetat und Ammoniumhydrogencarbonat und außerdem tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Als Verdünnungsmittel kommen bei der Durchführung des erfindungsgemäßen Verfahrens alle üblichen, inerten organischen Solventien in Betracht. Vorzugsweise verwendbar sind aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether wie Diethylether, Diisopropylether, Methyl-t-butylether, Methyl-t-amylether, Dioxan, Tetrahydrofuran, 1,2-Dimethoxyethan, 1,2-Diethoxyethan oder Anisol; Ketone, wie Aceton, Butanon, Methyl-isobutylketon oder Cyclohexanon; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester; Sulfoxide, wie Dimethylsulfoxid; Sulfone, wie Sulfolan.

Die Reaktionstemperaturen können bei der Durchführung des erfindungsgemäßen Verfahrens in einem größeren Bereich variiert werden. Im allgemeinen arbeitet man bei Temperaturen zwischen -20°C und +180°C, vorzugsweise bei Temperaturen zwischen 20°C und 130°C.

Das erfindungsgemäße Verfahren wird üblicherweise unter Normaldruck durchgeführt. Es ist jedoch auch möglich, unter erhöhtem oder vermindertem Druck zu arbeiten.

Bei der Durchführung des erfindungsgemäßen Verfahrens setzt man auf 1 Mol an 1,2,3-Thiadiazolcarbonsäure-halogenid der Formel (II) im allgemeinen 0,5 bis 10 Mol, vorzugsweise 0,8 bis 3 Mol an (Thio)Alkohol der Formel (III) ein. Die Aufarbeitung erfolgt nach üblichen Methoden (vgl. auch die Herstellungsbeispiele).

Die 1,2,3-Thiadiazolcarbonsäure(thio)ester der Formel (I) können in Säureadditions-Salze und Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerten organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, Isolieren und gegebenenfalls durch Umkristallisation reinigen.

Die erfindungsgemäßen Wirkstoffe weisen eine starke resistenz-induzierende Wirkung in Pflanzen auf. Sie eignen sich daher zur Erzeugung von Resistenz in Pflanzen gegen Befall durch unerwünschte Mikroorganismen.

Unter resistenzinduzierenden Stoffen sind im vorliegenden Zusammenhang solche Substanzen zu verstehen, die in der Lage sind, das Abwehrsystem von Pflanzen so zu stimulieren, daß die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um in Pflanzen innerhalb eines gewissen Zeitraumes nach der Behandlung Resistenz gegen den Befall durch die genannten Schaderreger zu erzeugen. Der Zeitraum, innerhalb dessen Resistenz herbeigeführt wird, erstreckt sich im allgemeinen von 1 bis 10 Tage, vorzugsweise 1 bis 7 Tage nach der Behandlung der Pflanzen mit den Wirkstoffen

Die erfindungsgemäßen Wirkstoffe weisen neben der resistenzinduzierenden Wirkung auch eine starke mikrobizide Wirkung auf und werden auch zur direkten Bekämpfung von unerwünschten Mikroorganismen praktisch eingesetzt. Die Wirkstoffe sind für den Gebrauch als Pflanzenschutzmittel, insbesondere als Fungizide geeignet.

Zu den unerwünschten Mikroorganismen im Pflanzenschutz gehören Pilze, wie Plasmodiophoromycetes, Oomycetes, Chytridiomycetes, Zygomycetes, Ascomycetes, Basidiomycetes, Deuteromycetes und ferner Bakterien, wie Pseudomonadaceae, Rhizobiaceae, Enterobacteriaceae, Corynebacteriaceae und Streptomycetaceae.

Beispielhaft aber nicht begrenzend seien einige Erreger von pilzlichen und bakteriellen Erkrankungen, die unter die oben aufgezählten Oberbegriffe fallen, genannt:
Xanthomonas-Arten, wie beispielsweise Xanthomonas campestris pv. oryzae;
Pseudomonas-Arten, wie beispielsweise Pseudomonas syringae pv. lachrymans; Erwinia-Arten, wie beispielsweise Erwinia amylovora;
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Bremia-Arten, wie beispielsweise Bremia lactucae;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea (Konidienform: Drechslera, Syn: Helminthosporium);
Cochliobolus-Arten, wie beispielsweise Cochliobolus sativus (Konidienform: Drechslera, Syn: Helminthosporium);
Uromyces-Arten, wie beispielsweise Uromyces appendiculatus;
Puccinia-Arten, wie beispielsweise Puccinia recondita;
Sclerotinia-Arten, wie beispielsweise Sclerotinia sclerotiorum;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besonders gutem Erfolg zur Bekämpfung von Reiskrankheiten, wie Pyricularia-Arten, sowie mit gutem Erfolg auch gegen Getreidekrankheiten, wie echten Mehltau, einsetzen.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel. Als Emulgier und/oder schaumerzeugende Mittel kommen in Frage: z.B. nichtionogene und anionische Emulgatoren, wie Polyoxyethylen-Fettsäureester, Polyoxyethylen-Fettalkoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen z.B. folgende Verbindungen in Frage:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thiazol-5-carboxanilid; 2,6-Dichloro-N-(4-trifluoromethylbenzyl)-benzamid; (E)-2-Methoxyimino-N-methyl-2-(2-phenoxyphenyl)-acetamid; 8-Hydroxychinolinsulfat; Methyl-(E)-2-{2-[6-(2-cyanophenoxy)-pyrimidin-4-yloxy]-phenyl}-3-methoxyacrylat; Methyl-(E)-methoximino [alpha-(o-tolyloxy)-o-tolyl]-acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodine, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropimorph, Fentinacetat, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan,
Kasugamycin, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel-dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram

### Bakterizide:

Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, 4-Bromo-2-(4-chlorphenyl)-1-(ethoxymethyl)-5-(triflupromethyl)-1H-pyrrole-3-carbonitrile, Bendiocarb, Benfuracarb, Bensultap, Betacyfluthrin, Bifenthrin, BPMC, Brofenprox, Bromophos A, Bufencarb, Buprofezin, Butocarboxim, Butylpyridaben,
Cadusafos, Carbaryl, Carbofuran, Carbophenothion, Carbosulfan, Cartap, Chloethocarb, Chlorethoxyfos, Chlorfenvinphos, Chlorfluazuron, Chlormephos, N-[(6-Chloro-3-pyridinyl)-methyl]-N'-cyano-N-methyl-ethanimidamide, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat,
   Dimethylvinphos, Dioxathion, Disulfoton,
Edifenphos, Emamectin, Esfenvalerat, Ethiofencarb, Ethion, Ethofenprox, Ethoprophos, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Fluazuron ,Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivermectin,
Lambda-cyhalothrin, Lufenuron,
Malathion, Mecarbam, Mevinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, Nitenpyram
Omethoat, Oxamyl, Oxydemethon M, Oxydeprofos,
Parathion A, Parathion M, Permethrin, Phenthoat, Phorat, Phosalon, Phosmet, Phosphamidon, Phoxim, Pirimicarb, Pirimiphos M, Pirimiphos A, Profenophos, Promecarb, Propaphos, Propoxur, Prothiophos, Prothoat, Pymetrozin, Pyrachlophos, Pyridaphenthion, Pyresmethrin, Pyrethrum, Pyridaben, Pyrimidifen, Pyriproxifen,
Quinalphos,
Salithion, Sebufos, Silafluofen, Sulfotep, Sulprofos,
Tebufenozide, Tebufenpyrad, Tebupirimiphos, Teflubenzuron, Tefluthrin, Temephos, Terbam, Terbufos, Tetrachlorvinphos, Thiafenox, Thiodicarb, Thiofanöx, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, Zetamethrin.

Auch eine Mischung mit anderen bekannten Wirkstoffen, wie Herbiziden oder mit Düngemitteln und Wachstumsregulatoren ist möglich.

Die erfindungsgemäßen Wirkstoffe können als solche, in Form ihrer handelsüblichen Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verschäumen, Bestreichen usw. Es ist ferner möglich, die Wirkstoffe nach dem Ultra-Low-Volume-Verfahren auszubringen oder die Wirkstoffzubereitung oder der Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001 Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

Die erfindungsgemäßen Verbindungen zeigen auch eine Wirkung gegen tierische Schädlinge, insbesondere gegen Blattinsekten.

Die erfindungsgemäßen Wirkstoffe wirken nicht nur gegen Pflanzen-, Hygiene- und Vorratsschädlinge, sondern auch auf dem veterinärmedizinischen Sektor gegen tierische Parasiten (Ektoparasiten) wie Schildzecken, Lederzecken, Räudemilben, Laufmilben, Fliegen (stechend und leckend), parasitierende Fliegenlarven, Läpse, Haarlinge, Federlinge und Flöhe. Zu diesen Parasiten gehören:

Aus der Ordnung der Anoplurida z.B. Haematopinus spp., Linognathus spp., Pediculus spp., Phtirus spp., Solenopotes spp..

Aus der Ordnung der Mallophagida und den Unterordnungen Amblycerina sowie Ischnocerina z.B. Trimenopon spp., Menopon spp., Trinoton spp., Bovicola spp., Werneckiella spp., Lepikentron spp., Damalina spp., Trichodectes spp., Felicola spp..

Aus der Ordnung Diptera und den Unterordnungen Nematocerina sowie Brachycerina z.B. Aedes spp., Anopheles spp., Culex spp., Simulium spp., Eusimulium spp., Phlebotomus spp., Lutzomyia spp., Culicoides spp., Chrysops spp., Hybomitra spp., Atylotus spp., Tabanus spp., Haematopota spp:, Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp..

Aus der Ordnung der Siphonapterida z.B. Pulex spp., Ctenocephalides spp., Xenopsylla spp., Ceratophyllus spp..

Aus der Ordnung der Heteropterida z.B. Cimex spp., Triatoma spp., Rhodnius spp. Panstrongylus spp..

Aus der Ordnung der Blattarida z.B. Blatta orientalis, Periplaneta americana, Blattela germanica, Supella spp..

Aus der Unterklasse der Acaria (Acarida) und den Ordnungen der Meta- sowie Mesostigmata z.B. Argas spp., Ornithodorus spp., Otabius spp., Ixodes spp., Amblyomma spp., Boophilus spp., Dermacentor spp., Haemaphysalis spp., Hyalomma spp., Rhipicephalus spp., Dermanyssus spp., Raillietia spp., Pneumonyssus spp., Sternostoma spp., Varroa spp..

Aus der Ordnung der Actinedida (Prostigmata) und Acaridida (Astigmata) z.B. Acarapis spp., Cheyletiella spp., Ornithocheyletia spp., Myobia spp., Psorergates spp., Demodex spp., Trombicula spp., Listrophorus spp., Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

Beispielsweise zeigen die erfindungsgemäßen Stoffe eine hervorragende Wirksamkeit gegen Musca domestica.

Die erfindungsgemäßen Wirkstoffe eignen sich auch zur Bekämpfung von Arthropoden, die landwirtschaftliche Nutztiere, wie z.B. Rinder, Schafe, Ziegen, Pferde, Schweine, Esel, Kamele, Büffel, Kaninchen, Hühner, Puten, Enten, Gänse, Bienen, sonstige Haustiere wie z.B. Hunde, Katzen, Stubenvögel, Aquarienfische sowie sogenannte Versuchstiere, wie z.B. Hamster, Meerschweinchen, Ratten und Mäuse befallen. Durch die Bekämpfung dieser Arthropoden sollen Todesfälle und Leistungsminderungen (bei Fleisch, Milch, Wolle, Häuten, Eiern, Honig usw.) vermindert werden, so daß durch den Einsatz der erfindungsgemäßen Wirkstoffe eine wirtschaftlichere und einfachere Tierhaltung möglich ist

Die Anwendung der erfindungsgemäßen Wirkstoffe geschieht im Veterinärsektor in bekannter Weise durch enterale Verabreichung, beispielsweise in Form von Tabletten, Kapseln, Tränken, Drenchen, Granulaten, Pasten, Boli, des feed-through-Verfahrens, von Zäpfchen, durch parenterale Verabreichung, wie zum Beispiel durch Injektionen (intramuskulär, subcutan, intravenös, intraperitonal u.a.), Implantate, durch nasale Applikation, durch dermale Anwendung, beispielsweise in Form des Tauchens oder Badens (Dippen), Sprühens (Spray), Aufgießens (Pour-on und Spot-on), des Waschens, des Einpuderns sowie mit Hilfe von wirkstoffhaltigen Formkörpern, wie Halsbändern, Ohrmarken, Schwanzmarken, Gliedmaßenbändern, Halftern, Markierungsvorrichtungen usw.

Bei der Anwendung für Vieh, Geflügel, Haustiere etc. kann man die erfindungsgemäßen Wirkstoffe als Formulierungen (beispielsweise Pulver, Emulsionen, fließfähige Mittel), die die Wirkstoffe in einer Menge von 1 bis 80 Gew.-% enthalten, direkt oder nach 100 bis 10 000-facher Verdünnung anwenden oder sie als chemisches Bad verwenden.

Die Herstellung und die Verwendung der erfindungsgemäßen Wirkstoffe gehen aus den nachfolgenden Beispielen hervor.

### Herstellungsbeispiele

### Beispiel 1:

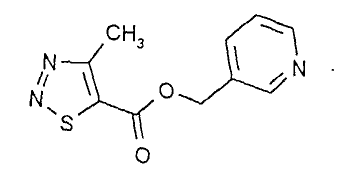

Eine Lösung von 30,5 g (0,28 Mol) 3-Hydroxymethylpyridin und 40 ml Triethylamin in 150 ml Toluol wird mit 40 g (0,246 Mol) 4-Methyl-1,2,3-thiadiazol-5-carbonsäurechlorid versetzt und 18 Stunden bei 100°C gerührt. Nach dem Abkühlen wird die Reaktionsmischung mit Wasser versetzt, die organische Phase abgetrennt, über Magnesiumsulfat getrocknet und eingedampft. Das so erhaltene Öl kristallisiert langsam aus. Man erhält 45,5 g (79 % der Theorie) an 4-Methyl-1,2,3-thiadiazol-5-carbonsäure-pyrid-3-ylmethylester vom Schmelzpunkt 48°C.

Nach der zuvor angegebenen Methode werden auch die in der folgenden Tabelle aufgeführten Stoffe der Formel (I) hergestellt.

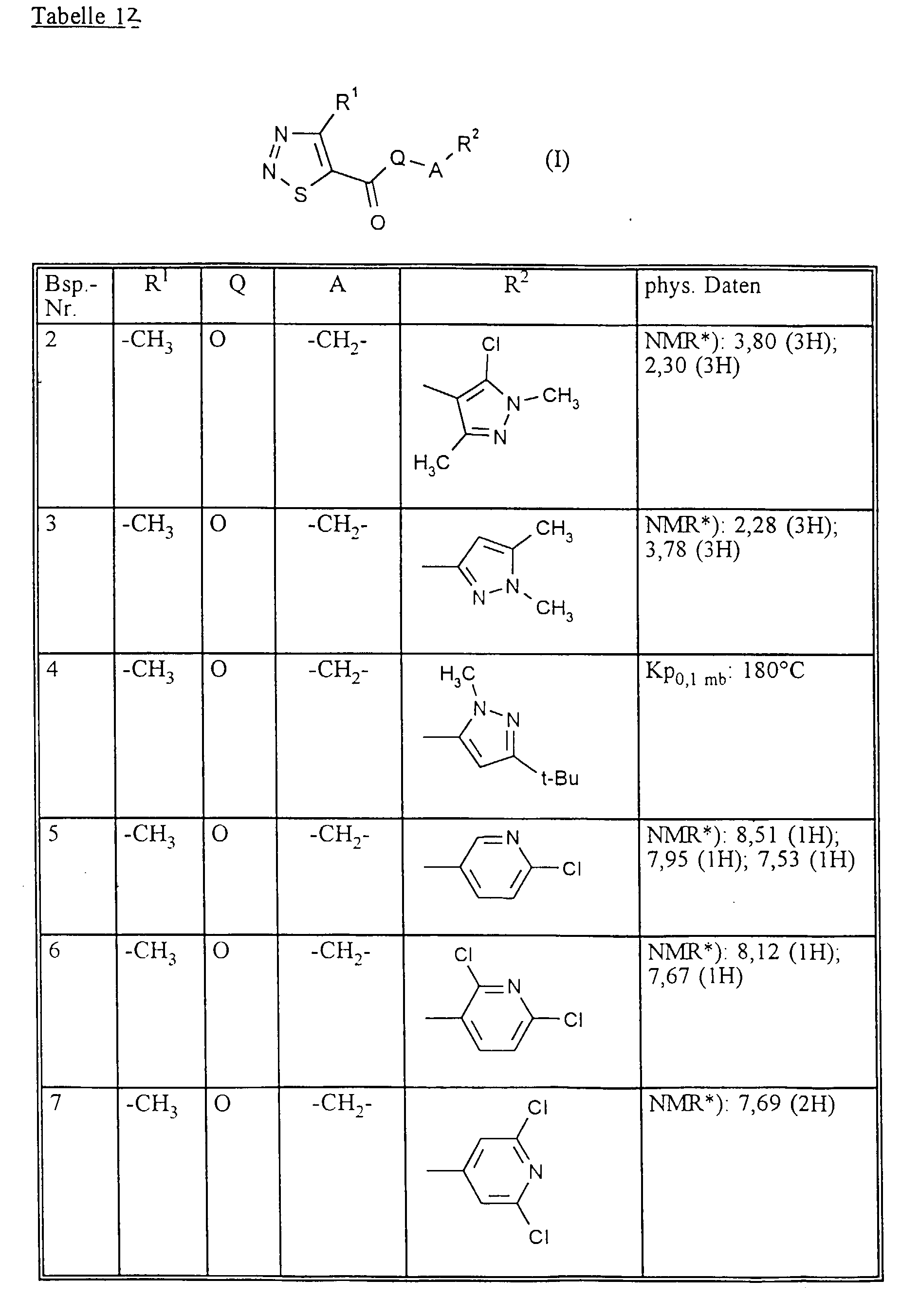

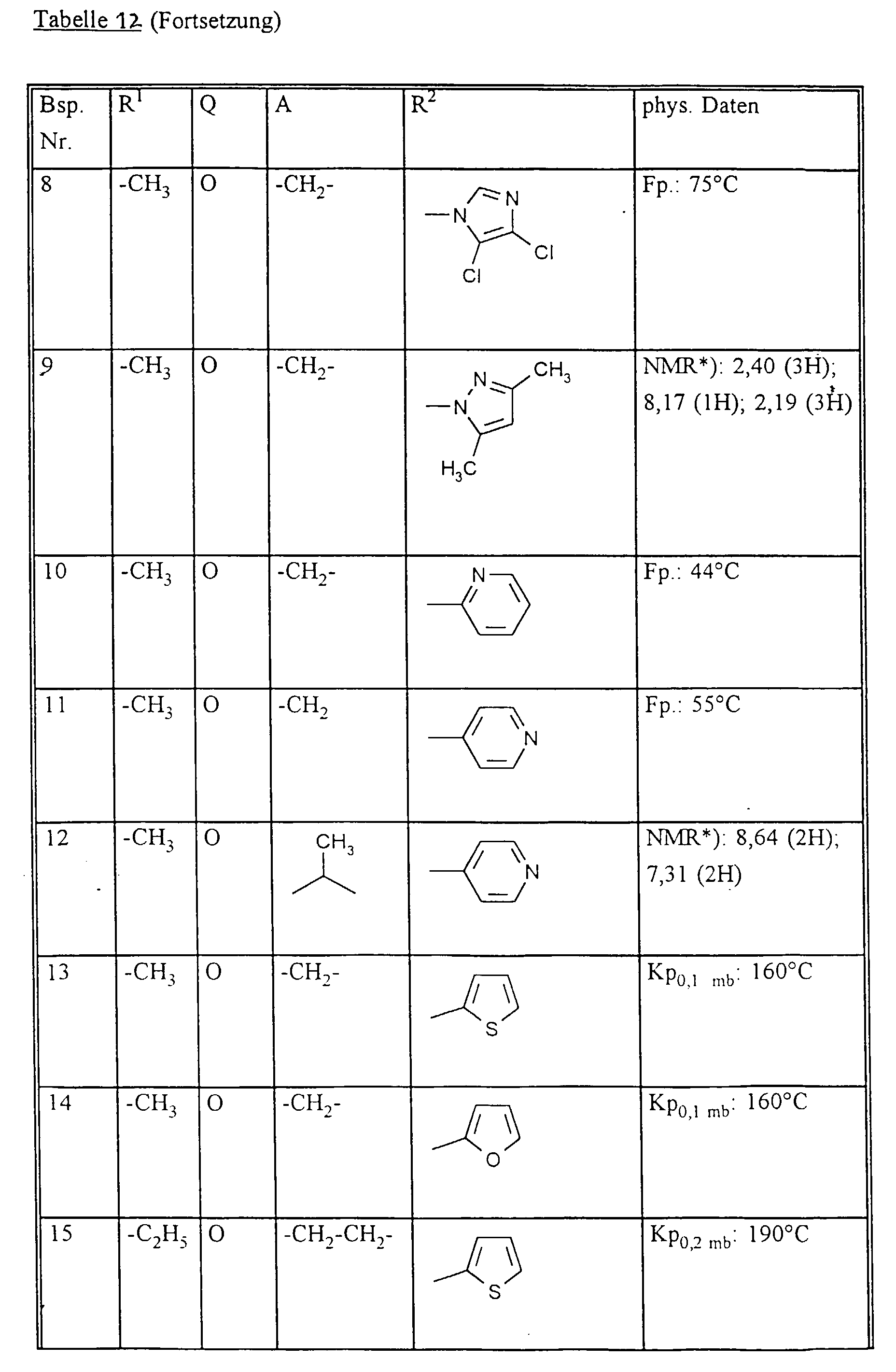

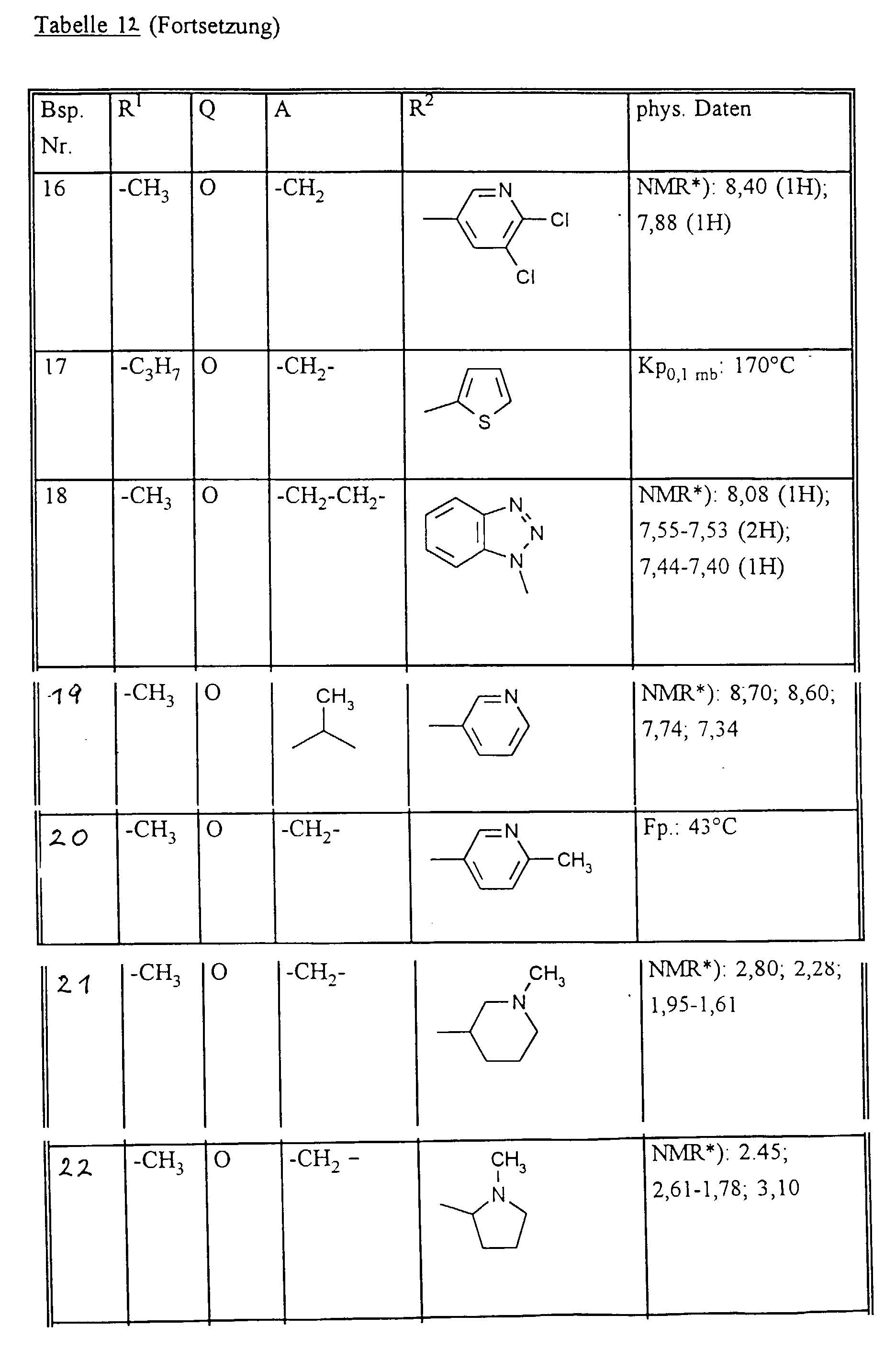

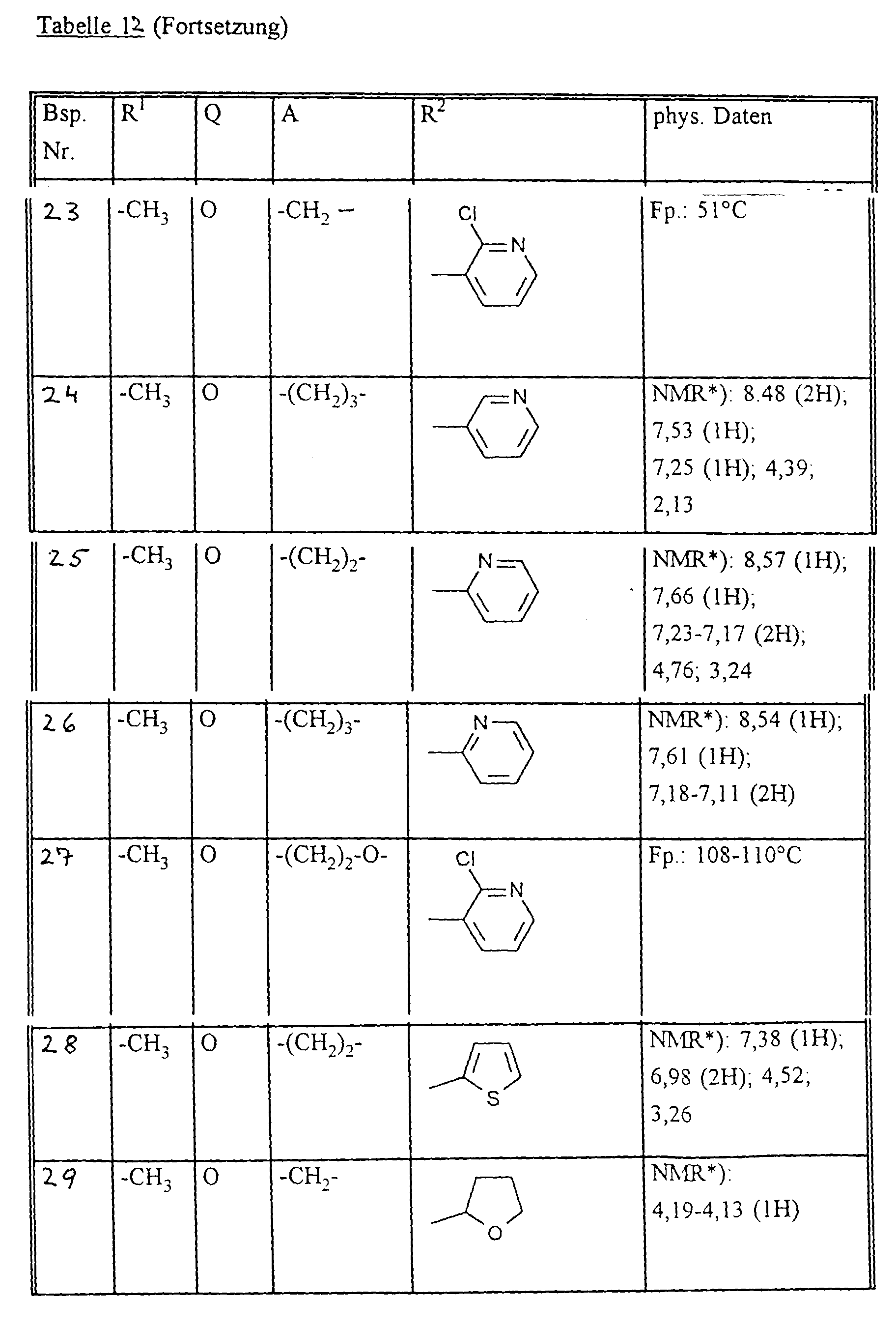

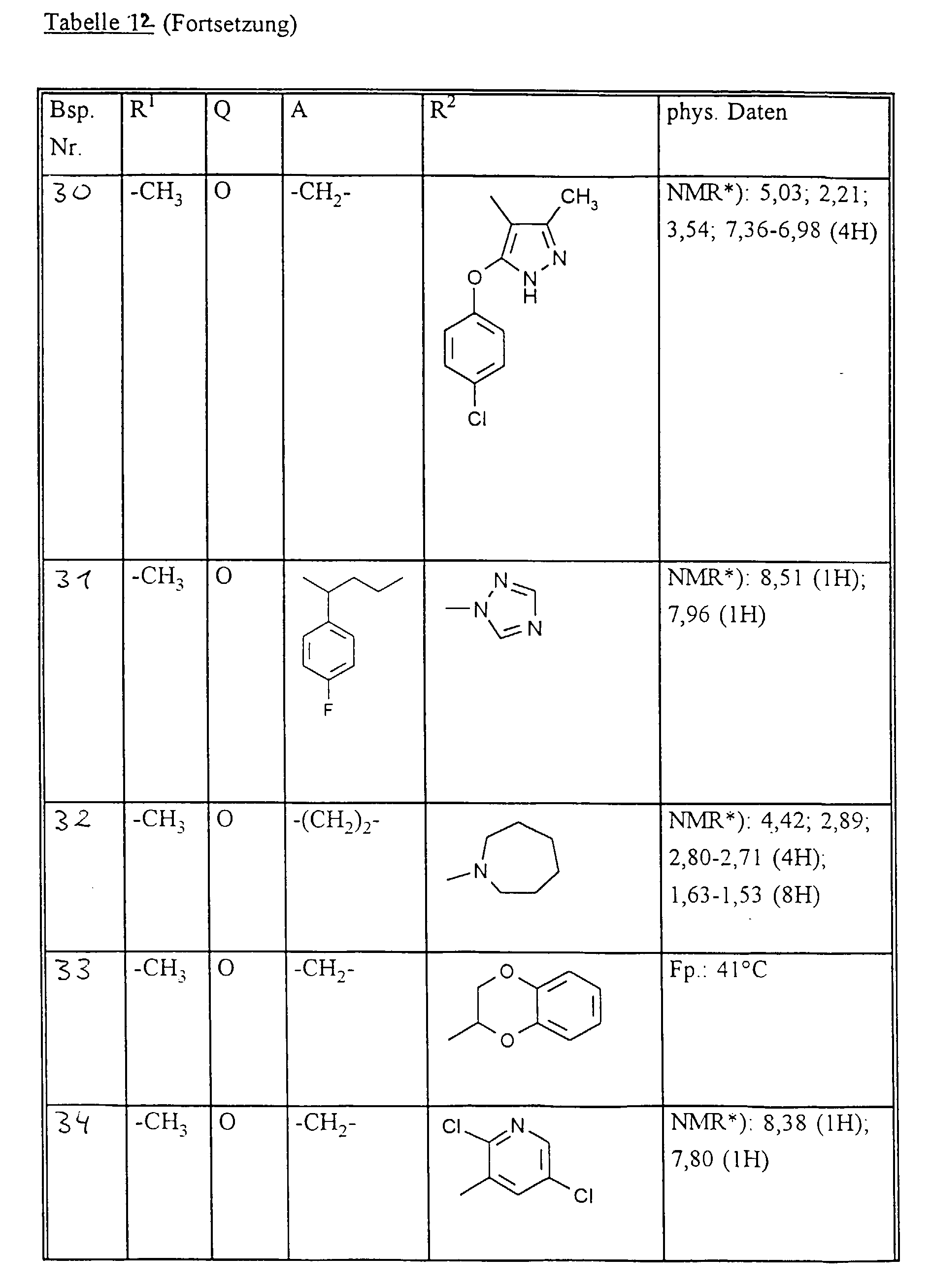

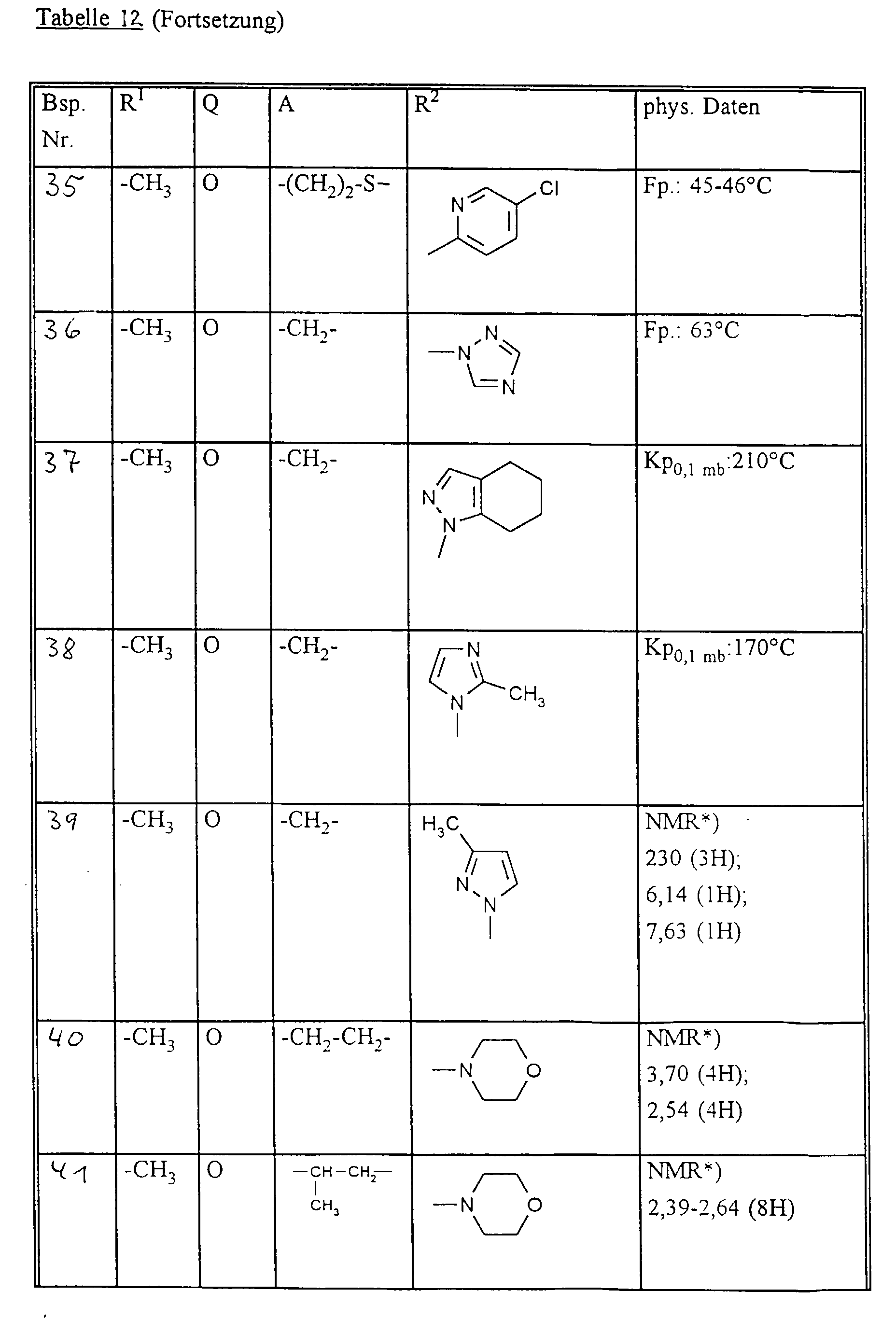

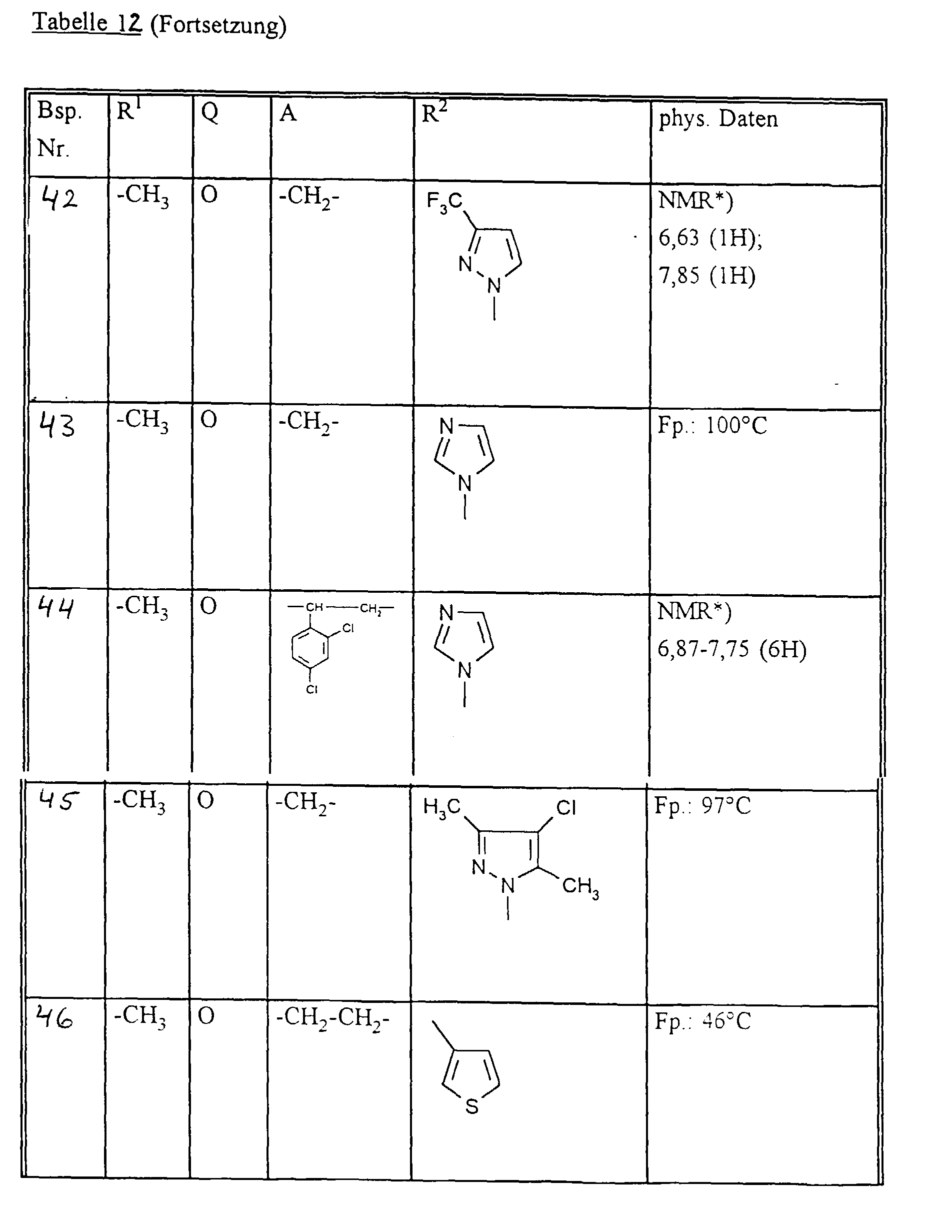

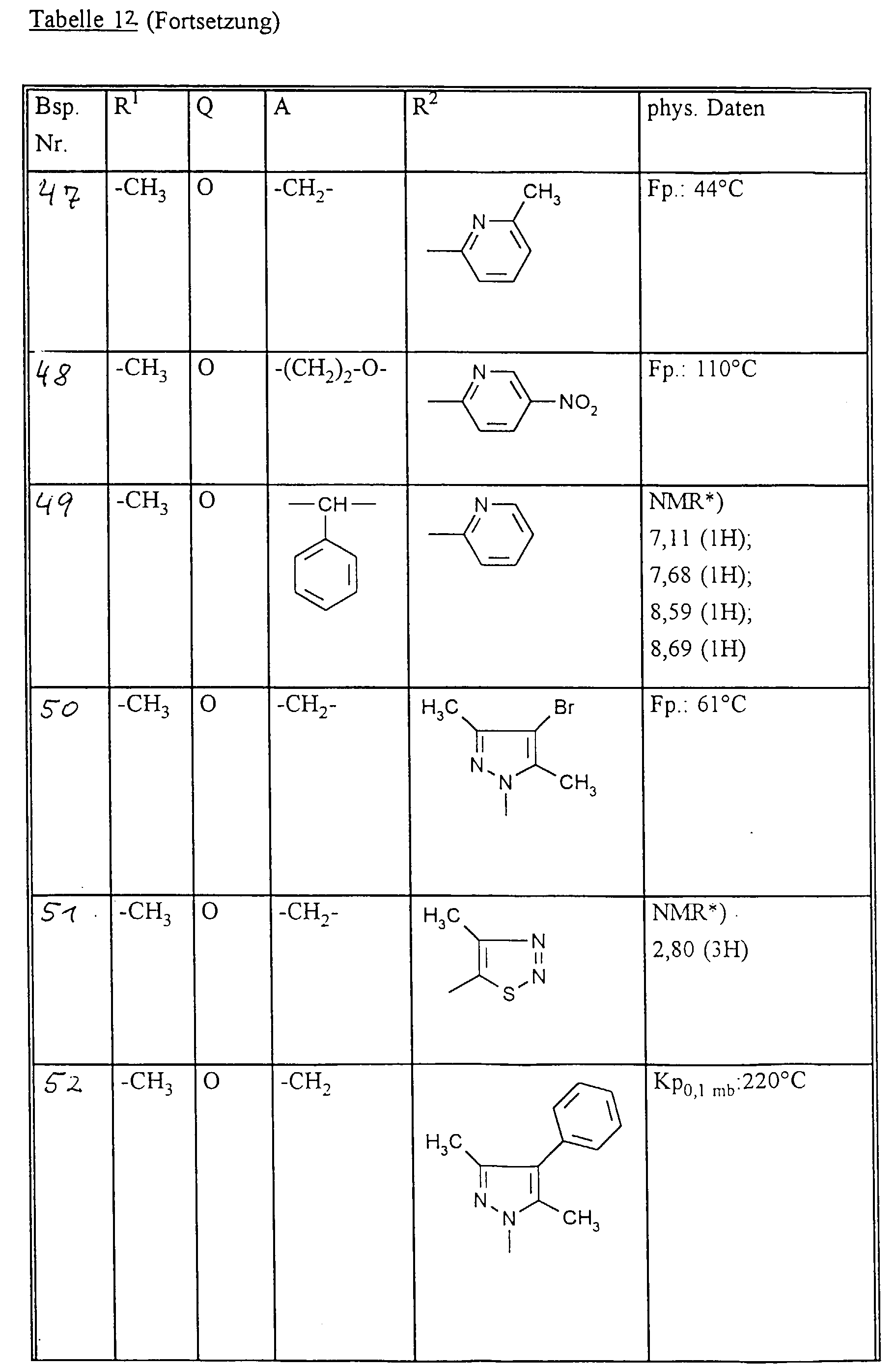

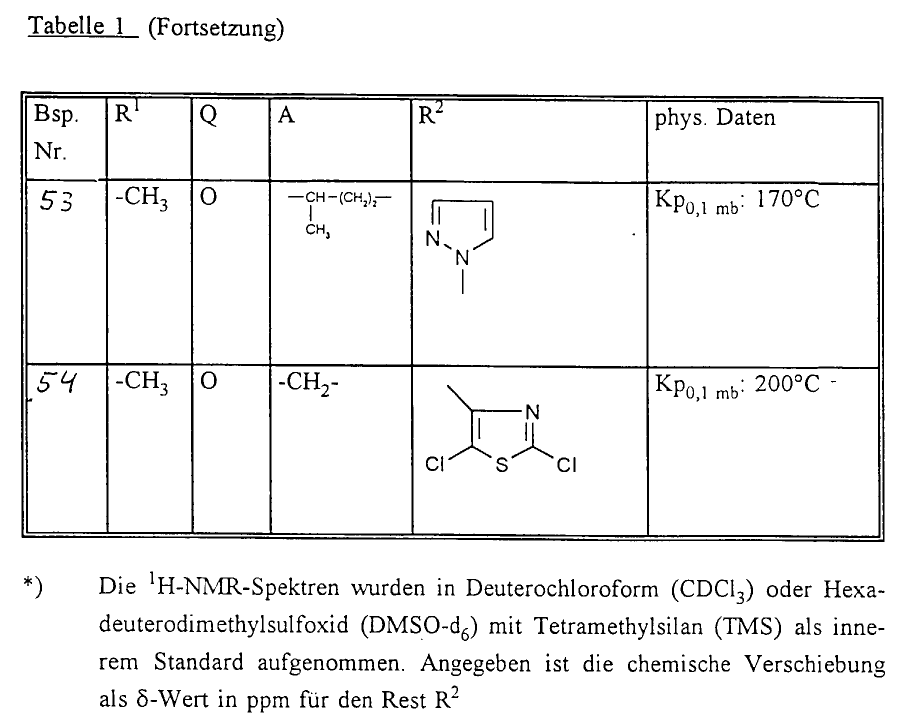

### Verwendungsbeispiele:

### Beispiel A

| Pyricularia-Test (Reis) / protektiv | |
|---|---|
| Lösungsmittel | 12,5 Gewichtsteile Aceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf protektive Wirksamkeit bespritzt man junge Reispflanzen mit der Wirkstoffzubereitung bis zur Tropfnässe. 5 Tage nach der Behandlung werden die Pflanzen mit einer wäßrigen Sporensuspension von Pyricularia oryzae inokuliert. Anschließend werden die Pflanzen in einem Gewächshaus bei 100% rel. Luftfeuchtigkeit und 25°C aufgestellt.

4 Tage nach der Inokulation erfolgt die Auswertung des Krankheitsbefalls. Dabei bedeutet 0% einen Wirkungsgrad, der demjenigen der Kontrolle entspricht, während ein Wirkungsgrad von 100% bedeutet, daß kein Befall beobachtet wird.

In diesem Test zeigen die in den Beispielen 1, 2 und 4 offenbarten erfindungsgemäßen Stoffe bei einer Wirkstoffkonzentration in der Spritzflüssigkeit von 0,0125 Gew.-% einen Wirkungsgrad von 70 % oder mehr.

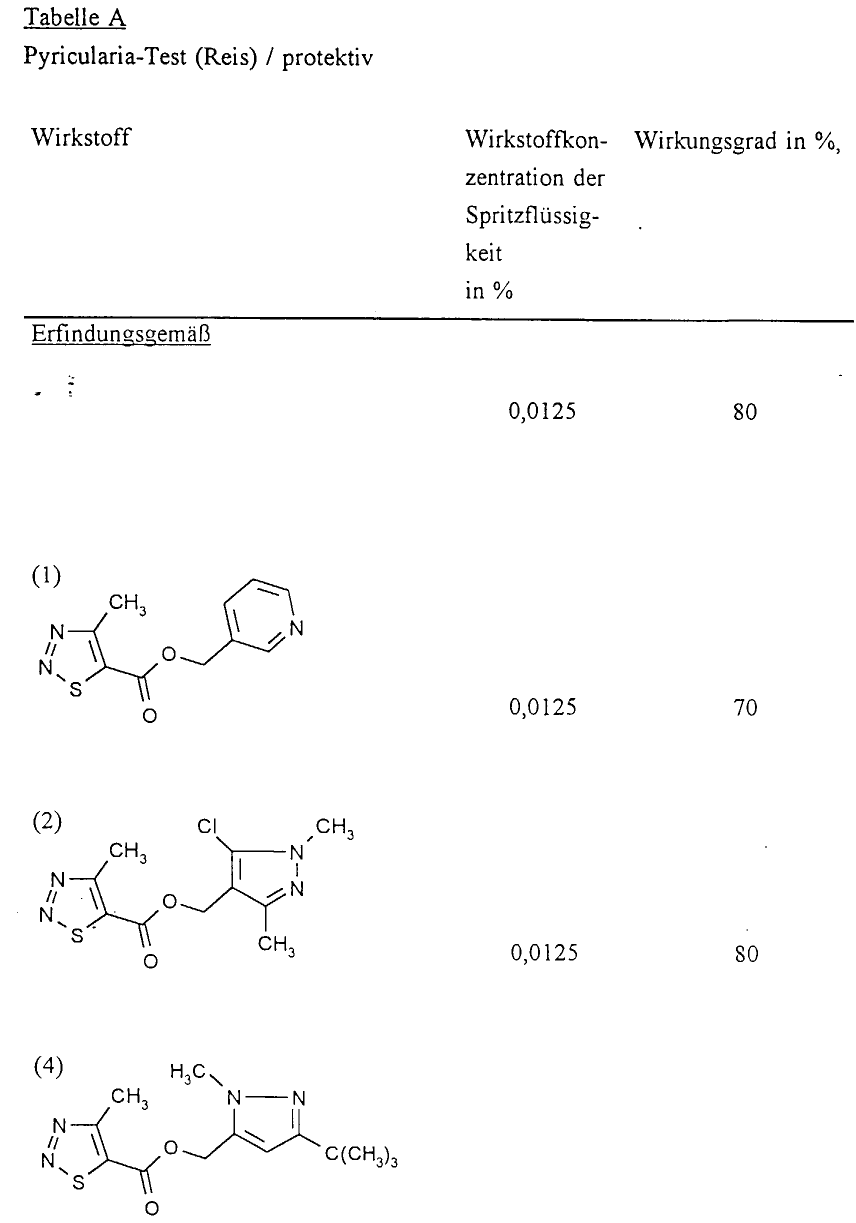

### Beispiel B

| Pyricularia-Test (Reis) / systemisch | |
|---|---|
| Lösungsmittel | 12,5 Gewichtsteile Aceton |
| Emulgator | 0,3 Gewichtsteile Alkylarylpolyglykolether |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser und der angegebenen Menge Emulgator auf die gewünschte Konzentration.

Zur Prüfung auf systemische Eigenschaften werden 40 ml der Wirkstoffzubereitung auf Einheitserde gegossen, in der junge Reispflanzen angezogen wurden. 7 Tage nach der Behandlung werden die Pflanzen mit einer wäßrigen Sporensuspension von Pyricularia oryzae inokuliert. Danach verbleiben die Pflanzen in einem Gewächshaus bei einer Temperatur von 25°C und einer rel. Luftfeuchtigkeit von 100% bis zur Auswertung.

4 Tage nach der Inokulation erfolgt die Auswertung des Krankheitsbefalls. Dabei bedeutet 0% einen Wirkungsgrad, der demjenigen der Kontrolle entspricht, während ein Wirkungsgrad von 100% bedeutet, daß kein Befall beobachtet wird.

In diesem Test zeigen die in den Beispielen 8, 9, 11 und 13 offenbarten erfindungsgemäßen Stoffe bei einer Konzentration von 100 mg Wirkstoff pro 100 cm² einen Wirkungsgrad von über 60 %.

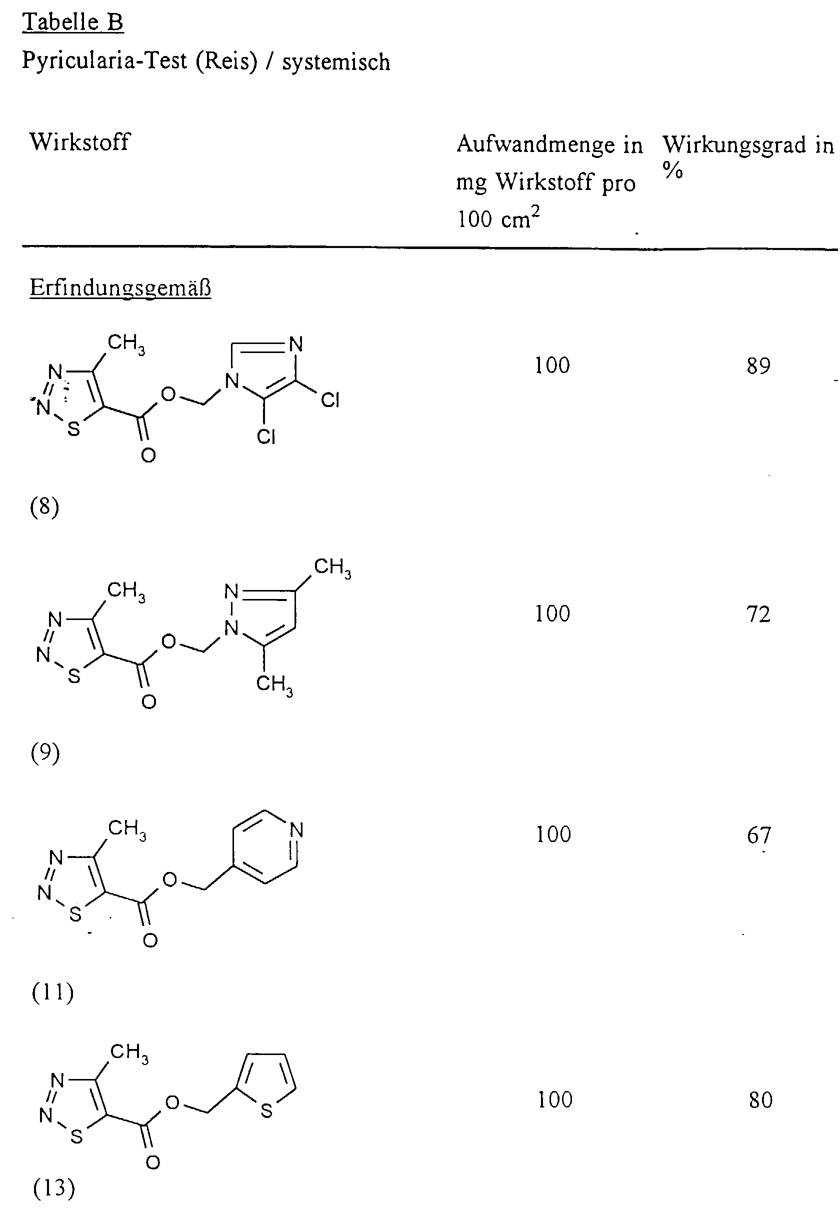

### Beispiel C

| Test mit Fliegen (Musca domestica) | |
|---|---|
| Testtiere | adulte Musca domestica, Stamm Reichswald (OP, SP, Carbamat-resistent) |
| Lösungsmittel | 35 Gewichtsteile Ethylenglykolmonomethylether |
| Emulgator | 35 Gewichtsteile Nonylphenolpolyglykolether |

Zur Herstellung einer geeigneten Formulierung vermischt man drei Gewichtsteile Wirkstoff mit sieben Teilen des oben angegebenen Lösungsmittel-Emulgator-Gemisches und verdünnt das so erhaltene Emulsionskonzentrat mit Wasser auf die,jeweils gewünschte Konzentration.

2 ml dieser Wirkstoffzubereitung werden auf Filterpapierschalen (9,5 cm) pipettiert, die sich in Petrischalen entsprechender Größe befinden. Nach Trocknung der Filterscheiben werden 25 Testtiere in die Petrischale überführt und abgedeckt.

Nach 24 Stunden wird die Wirksamkeit der Wirkstoffzubereitung ermittelt. Dabei bedeutet 100%, daß alle Fliegen abgetötet wurden; 0% bedeutet, daß keine Fliegen abgetötet wurden.

In diesem Test zeigt der im Beispiel 10 offenbarte erfindungsgemäße Stoff bei einer Wirkstoffkonzentration von 1000 ppm einen Wirkungsgrad von 100 %.

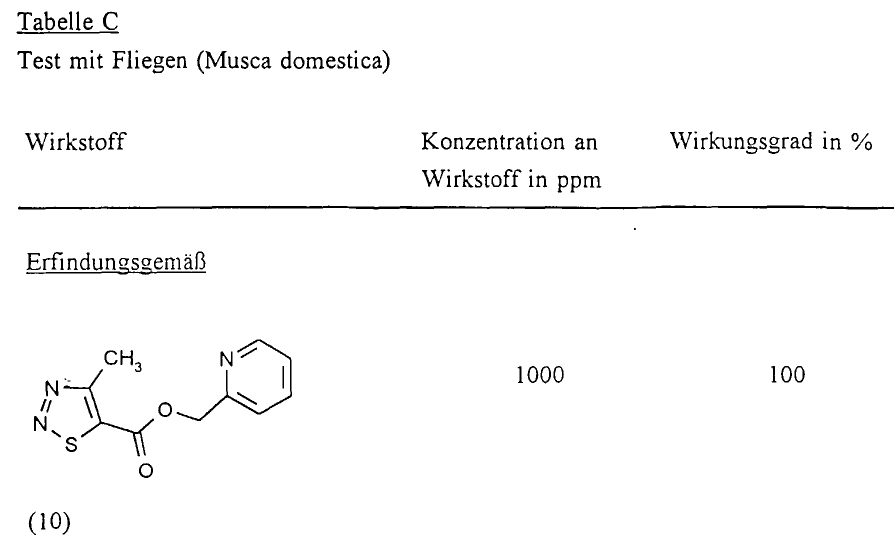

## Patentansprüche

1. 1,2,3-Thiadiazolcarbonsäure(thio)ester der Formel 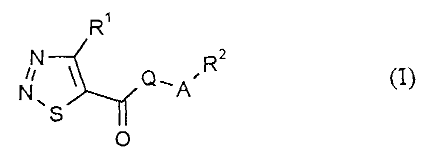 in welcher
A für Methylen, Ethan-1,1-diyl, Ethan-1,2-diyl, Propan-1,3-diyl, Butan-1,3-diyl, Ethen-1,2-diyl, Propen-1,3-diyl, Phenylmethan-1,1-diyl, 1-(2,4-dichloro-phenyl)-ethan-1,2-diyl steht,
oder für 1-Phenyl-propan-1,3-diyl steht, wobei der Rest R² an die 3-Position gebunden ist,
oder für 1-(4-Fluorphenyl)-propan-1,3-diyl steht, wobei der Rest R² an die 3-Position gebunden ist,
oder für die Gruppen
-CH₂-O-CH₂-, -CH₂-S-CH₂-, 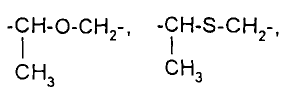 -CH=CH-CH₂-O-CH₂- und -CH=CH-CH₂-S-CH₂-, sowie für die Gruppen -CH₂-O-, -CH₂-CH₂-O-, -CH₂-S- und -CH₂-CH₂-S-
steht, wobei das Heteroatom jeweils mit dem Rest R² verbunden ist,
Q für Sauerstoff oder Schwefel steht,
R¹ für Wasserstoff, Methyl, Ethyl, n-Propyl, Isopropyl, n-Butyl, Trichlormethyl, Trifluormethyl, Fluormethyl, Difluormethyl oder für Phenyl oder Naphthyl, wobei jeder dieser beiden zuletzt genannten Reste einfach bis dreifach, gleichartig oder verschieden substituiert sein kann durch Fluor, Chlor, Brom, Iod, Cyano, Nitro, Methyl, Ethyl, Methoxy, Ethoxy, Trichlormethyl, Trifluormethyl und/oder Trifluormethoxy steht und
R² für Pyrrolyl, Furyl, Thienyl, Imidazolyl, Pyrazolyl, Triazolyl, Oxazolyl, Isoxazolyl, Thiazolyl, Isothiazolyl, Oxadiazolyl, Thiadiazolyl, Tetrazolyl, Pyridyl, Pyrimidyl, Pyridazinyl, Pyrazinyl, Triazinyl, Indolyl, Benzimidazolyl, Benzothiazolyl, Benzotriazolyl oder Benzodioxanyl, Oxetanyl, Tetrahydrofuryl, Perhydropyranyl, Pyrrolidinyl, Piperidinyl, Morpholinyl, Dioxolanyl, Dioxanyl, Hexahydroazepinyl, Chinuclidinyl, Benzofuranyl, Benzothienyl, Oxiranyl, Thiazinyl, Dihydrothiazinyl oder Tetrahydrothiazinyl steht, wobei diese Reste einfach bis dreifach, gleichartig oder verschieden substituiert sein können durch Fluor, Chlor, Brom, Iod, Cyano, Nitro, Amino, Hydroxy, Formyl, Carboxy, Carbamoyl, Thiocarbamoyl, Methyl, Ethyl, n-oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Trifluorethyl, Difluormethoxy, Trifluormethoxy, Difluorchlormethoxy, Trifluorethoxy, Difluormethylthio, Difluorchlormethylthio, Trifluormethylthio, Trifluormethylsulfinyl, Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl, Ethoximinoethyl, Phenyl, Chlorphenyl, Methylphenyl, Phenoxy, Chlorphenoxy, Fluorphenoxy, Methylphenoxy, Cyclopropyl, Cyclobutyl, Cyclopentyl, Cyclohexyl, Dioxolanyl, das seinerseits einfach oder zweifach durch Methyl substituiert sein kann, und/oder durch Dioxanyl, das seinerseits einfach oder zweifach durch Methyl substituiert sein kann,
und/oder auch in der Weise substituiert sein können, daß jeweils zwei benachbarte Substituenten einen ankondensierten, carbocyclischen Ring mit fünf oder sechs Ringgliedern bilden, wobei der Carbocyclus seinerseits einfach bis vierfach durch Fluor, Chlor, Brom und/oder Methyl substituiert sein kann,
und/oder auch in der Weise substituiert sein können, daß jeweils zwei benachbarte Substituenten einen ankondensierten heterocyclischen Ring mit fünf oder sechs Ringgliedern und 1 oder 2 Sauerstoff-, Schwefel- und/oder Stickstoffatomen bilden, wobei der Heterocyclus seinerseits einfach oder zweifach durch Fluor, Chlor, Brom und/oder Methyl substituiert sein kann,
sowie deren Säureadditions-Salze und Metallsalz-Komplexe.

2. Verfahren zur Herstellung von 1,2,3-Thiadiazolcarbonsäure(thio)estern der Formel (I) gemäß Anspruch 1 sowie von deren Säureadditions-Salzen und Metallsalz-Komplexen, dadurch gekennzeichnet, daß man 1,2,3-Thiadiazol-carbonsäure-halogenide der Formel 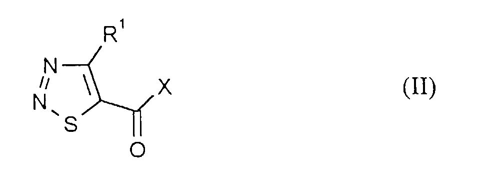 in welcher
R¹ die oben angegebene Bedeutung hat und
X für Halogen steht,
mit (Thio)Alkoholen der Formel
H-Q-A-R² (III)
in welcher
A, Q und R² die oben angegebenen Bedeutungen haben,
gegebenenfalls in Gegenwart eines Säurebindemittels und gegebenenfalls in Gegenwart eines Verdünnungsmittels umsetzt und gegebenenfalls anschließend an die so erhaltenen Verbindungen der Formel (I) eine Säure oder ein Metallsalz addiert.

3. Mittel zum Schutz von Pflanzen gegen Befall durch unerwünschte Mikroorganismen und zur Bekämpfung tierischer Schädlinge, gekennzeichnet durch einen Gehalt an mindestens einem 1,2,3-Thiadiazolcarbonsäure(thio)ester der Formel (I) gemäß Anspruch 1 bzw. einem Säure-additions-Salz oder Metallsalz-Komplex eines 1,2,3-Thiadiazolcarbonsäure(thio)esters der Formel (I).

4. Verwendung von 1,2,3-Thiadiazolcarbonsäure(thio)estern der Formel (I) gemäß Anspruch 1 bzw. von deren Säureadditions-Salzen und Metallsalz-Komplexen zum Schutz von Pflanzen gegen Befall durch unerwünschte Mikroorganismen und tierische Schädlinge.

5. Verfahren zum Schutz von Pflanzen gegen Befall durch unerwünschte Mikroorganismen und tierische Schädlinge, dadurch gekennzeichnet, daß man 1,2,3-Thiadiazolcarbonsäure(thio)ester der Formel (I) gemäß Anspruch 1 bzw. deren Säureadditions-Salze oder Metallsalz-Komplexe auf die unerwünschten Mikroorganismen oder tierischen Schädlinge und/oder deren Lebensraum ausbringt.

6. Verfahren zur Herstellung von Mitteln zum Schutz von Pflanzen gegen Befall durch unerwünschte Mikroorganismen und zur Bekämpfung tierischer Schädlinge, dadurch gekennzeichnet, daß man 1,2,3-Thiadiazolcarbonsäure(thio)ester der Formel (I) gemäß Anspruch 1 bzw. deren Säureadditions-Salze oder Metallsalz-Komplexe mit Streckmitteln und/oder oberflächenaktiven Stoffen vermischt.

## Claims

1. 1,2,3-Thiadiazolecarboxylic (thio)esters of the formula 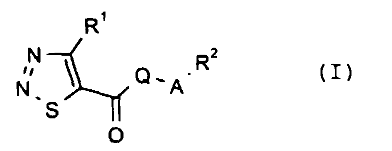 in which
A represents methylene, ethane-1,1-diyl, ethane-1,2-diyl, propane-1,3-diyl, butane-1,3-diyl, ethene-1,2-diyl, propene-1,3-diyl, phenylmethane-1,1-diyl, 1-(2,4-dichloro-phenyl)ethane-1,2-diyl,
or represents 1-phenyl-propane-1,3-diyl, where the radical R² is attached to the 3-position,
or represents 1-(4-fluorophenyl)-propane-1,3-diyl, where the radical R² is attached to the 3-position,
or represents the groups
-CH₂-O-CH₂-, -CH₂-S-CH₂-, 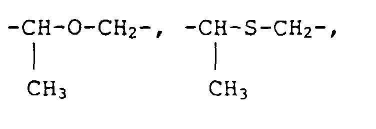 -CH=CH-CH₂-O-CH₂- and -CH=CH-CH₂-S-CH₂-, and also the groups -CH₂-O-, -CH₂-CH₂-O-, -CH₂-S- and -CH₂CH₂-S-,
where the heteroatom is in each case attached to the radical R²,
Q represents oxygen or sulphur,
R¹ represents hydrogen, methyl, ethyl, n-propyl, isopropyl, n-butyl, trichloromethyl, trifluoromethyl, fluoromethyl, difluoromethyl or represents phenyl or naphthyl, where each of these two last-mentioned radicals may be mono- to trisubstituted by identical or different radicals from the group consisting of fluorine, chlorine, bromine, iodine, cyano, nitro, methyl, ethyl, methoxy, ethoxy, trichloromethyl, trifluoromethyl and/or trifluoromethoxy and
R² represents pyrrolyl, furyl, thienyl, imidazolyl, pyrazolyl, triazolyl, oxazolyl, isoxazolyl, thiazolyl, isothiazolyl, oxadiazolyl, thiadiazolyl, tetrazolyl, pyridyl, pyrimidyl, pyridazinyl, pyrazinyl, triazinyl, indolyl, benzimidazolyl, benzothiazolyl, benzotriazolyl or benzodioxanyl, oxetanyl, tetrahydrofuryl, perhydropyranyl, pyrrolidinyl, piperidinyl, morpholinyl, dioxolanyl, dioxanyl, hexahydroazepinyl, chinuclidinyl, benzofuranyl, benzothienyl, oxiranyl, thiazinyl, dihydrothiazinyl or tetrahydrothiazinyl, where these radicals may be mono- to trisubstituted by identical or different radicals from the group consisting of fluorine, chlorine, bromine, iodine, cyano, nitro, amino, hydroxy, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, trifluoroethyl, difluoromethoxy, trifluoromethoxy, difluorochloromethoxy, trifluoroethoxy, difluoromethylthio, difluorochloromethylthio, trifluoromethylthio, trifluoromethylsulphinyl, trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroximinomethyl, hydroximinioethyl, methoximinomethyl, ethoximinomethyl, methoximinoethyl, ethoximinoethyl, phenyl, chlorophenyl, methylphenyl, phenoxy, chlorophenoxy, fluorophenoxy, methylphenoxy, cyclopropyl, cyclobutyl, cyclopentyl, cyclohexyl, dioxolanyl, which for its part may be mono- or disubstituted by methyl and/or by dioxanyl, which for its part may be mono- or disubstituted by methyl,
and/or may also be substituted such that in each case two adjacent substituents form a fused-on carbocyclic ring having five or six ring members where the carbocycle for its part may be mono- to tetrasubstituted by fluorine, chlorine, bromine and/or methyl,
and/or may also be substituted such that in each case two adjacent substituents form a fused-on heterocyclic ring having five or six ring members and 1 or 2 oxygen, sulphur and/or nitrogen atoms, where the heterocycle for its part may be mono- or disubstituted by fluorine, chlorine, bromine and/or methyl,
and their acid addition salts and metal salt complexes.

2. Process for preparing 1,2,3-thiadiazolecarboxylic (thio)esters of the formula (I) according to Claim 1 and acid addition salts and metal salt complexes thereof, characterized in that 1,2,3-thiadiazolecarbonyl halides of the formula 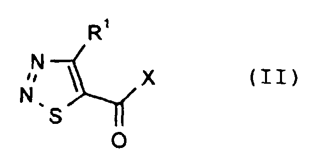 in which
R¹ is as defined above and
X represents halogen
are reacted with (thio)alcohols of the formula
H-Q-A-R² (III)
in which
A, Q and R² are as defined above,
if appropriate in the presence of an acid binder and if appropriate in the presence of a diluent, and, if appropriate, an acid or a metal salt is then added to the resulting compounds of the formula (I).

3. Composition for protecting plants against attack by unwanted microorganisms and for controlling animal pests, characterized in that it comprises at least one 1,2,3-thiadiazolecarboxylic (thio)ester of the formula (I) according to Claim 1 or one acid addition salt or metal salt complex of a 1,2,3-thiadiazolecarboxylic (thio)ester of the formula (I).

4. Use of 1,2,3-thiadiazolecarboxylic (thio)esters of the formula (I) according to Claim 1 or of acid addition salts and metal salt complexes thereof for protecting plants against attack by unwanted microorganisms and animal pests.

5. Method for protecting plants against attack by unwanted microorganisms and animal pests, characterized in that 1,2,3-thiadiazolecarboxylic (thio)esters of the formula (I) according to Claim 1 or acid addition salts or metal salt complexes thereof are applied to the unwanted microorganisms or animal pests and/or their habitat.

6. Process for preparing compositions for protecting plants against attack by unwanted microorganisms and for controlling animal pests, characterized in that 1,2,3-thiadiazolecarboxylic (thio)esters of the formula (I) according to Claim 1 or acid addition salts or metal salt complexes thereof are mixed with extenders and/or surfactants.

## Revendications

1. (Thio)esters d'acide 1,2,3-thiadiazolcarboxylique de formule 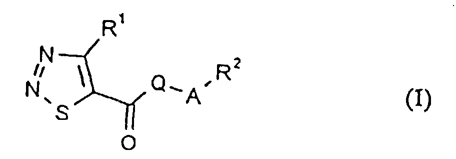 dans laquelle
A est un groupe méthylène, éthane-1,1-diyle, éthane-1,2-diyle, propane-1,3-diyle, butane-1,3-diyle, éthène-1,2-diyle, propène-1,3-diyle, phénylméthane-1,1-diyle, 1-(2,4-dichlorophényl)éthane-1,2-diyle,
ou un groupe 1-phénylpropane-1,3-diyle, le reste R² étant alors lié à la position 3, ou le groupe 1-(4-fluorophényl)propane-1,3-diyle, le reste R² étant alors lié à la position 3,
ou bien les groupes
-CH₂-O-CH₂-, -CH₂-S-CH₂-, 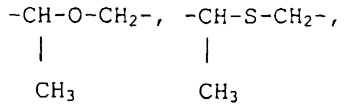 -CH=CH-CH₂-O-CH₂- et -CH=CH-CH₂-S-CH₂-, de même que les groupes -CH₂-O-, -CH₂-CH₂-O-, -CH₂-S- et -CH₂-CH₂-S-,
l'hétéroatome étant alors lié dans chaque cas au reste R²,
Q représente l'oxygène ou le soufre,
R¹ représente l'hydrogène, un reste méthyle, éthyle, n-propyle, isopropyle, n-butyle, trichlorométhyle, trifluorométhyle, fluorométhyle, difluorométhyle ou un reste phényle ou naphtyle, chacun des deux restes mentionnés en dernier lieu pouvant être substitué une à trois fois identiques ou différentes par du fluor, du chlore, du brome, de l'iode, un radical cyano, nitro, méthyle, éthyle, méthoxy, éthoxy, trichlorométhyle, trifluorométhyle et/ou trifluorométhoxy et
R² est un reste pyrrolyle, furyle, thiényle, imidazolyle, pyrazolyle, triazolyle, oxazolyle, isoxazolyle, thiazolyle, isothiazolyle, oxadiazolyle, thiadiazolyle, tétrazolyle, pyridyle, pyrimidyle, pyridazinyle, pyrazinyle, triazinyle, indolyle, benzimidazolyle, benzothiazolyle, benzotriazolyle ou benzodioxanyle, oxétanyle, tétrahydrofuryle, perhydropyrannyle, pyrrolidinyle, pipéridinyle, morpholinyle, dioxolanyle, dioxanyle, hexahydroazépinyle, quinuclidinyle, benzofurannyle, benzothiényle, oxiranyle, thiazinyle, dihydrothiazinyle ou tétrahydrothiazinyle, ces restes pouvant être substitués une à trois fois identiques ou différentes par du fluor, du chlore, du brome, de l'iode, un radical cyano, nitro, amino, hydroxy, formyle, carboxy, carbamoyle, thiocarbamoyle, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, trifluoréthyle, difluorométhoxy, trifluorométhoxy, difluorochlorométhoxy, trifluoréthoxy, difluorométhylthio, difluorochorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle hydroximinoéthyle, méthoximinométhyle, éthoximinométhyle, méthoximinoéthyle, éthoximinoéthyle, phényle, chlorophényle, méthylphényle, phénoxy, chlorophénoxy, fluorophénoxy, méthylphénoxy, cyclopropyle, cyclobutyle, cyclopentyle, cyclohexyle, dioxolanyle, qui peut de son côté être substitué une ou deux fois identiques ou différentes par un radical méthyle, et/ou par un radical dioxanyle qui peut lui-même être substitué une ou deux fois par un radical méthyle, et/ou peuvent aussi être substitués de façon telle que chaque fois deux substituants voisins forment un noyau carbocyclique condensé de cinq ou six atomes, le carbocycle pouvant lui-même être substitué une à quatre fois par du fluor, du chlore, du brome et/ou un radical méthyle,
et/ou peuvent aussi être substitués de façon telle que chaque fois deux substituants voisins forment un noyau hétérocyclique condensé ayant cinq ou six chaînons et 1 ou 2 atomes d'oxygène, de soufre et/ou d'azote, l'hétérocycle pouvant être substitué de son côté une ou deux fois par du fluor, du chlore, du brome et/ou un radical méthyle,
ainsi que leurs sels d'addition d'acides et leurs complexes formés avec des sels métalliques.

2. Procédé de production de (thio)esters d'acide 1,2,3-thiadiazolecarboxylique de formule (I) suivant la revendication 1 ainsi que de leurs sels d'addition d'acides et de leurs complexes formés avec des sels métalliques caractérisé en ce qu'on fait réagir des halogénures d'acide 1,2,3-thiadiazolecarboxylique de formule 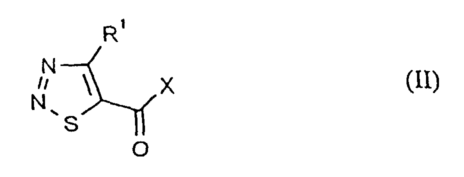 dans laquelle
R¹ a la définition indiquée ci-dessus et
X représente un halogène,
avec des (thio)alcools de formule
H-Q-A-R² (III)
dans laquelle
A, Q et R² ont les définitions indiquées ci-dessus,
le cas échéant en présence d'un accepteur d'acide et en présente éventuelle d'un diluant, après quoi on additionne ensuite, le cas échéant, un acide ou un sel métallique sur les composés de formule (I) ainsi obtenus.

3. Compositions pour protéger des plantes d'une attaque par des micro-organismes indésirables et pour combattre des parasites animaux, caractérisées par une teneur en au moins un (thio)ester d'acide 1,2,3-thiadiazolecarboxylique de formule (I) suivant la revendication 1 ou d'un sel d'addition d'acide ou d'un complexe de sel métallique d'un (thio)ester d'acide 1,2,3-thiadiazolecarboxylique de formule (I).

4. Utilisation de (thio)esters d'acide 1,2,3-thiadiazolecarboxylique de formule (I) suivant la revendication 1 ou de leurs sels d'addition d'acides et leurs complexes de sels métalliques pour la protection de plantes contre une attaque par des micro-organismes indésirables et des parasites animaux.

5. Procédé pour protéger des plantes d'une attaque par des micro-organismes indésirables et des parasites animaux, caractérisé en ce qu'on répand des (thio)esters d'acide 1,2,3-thiadiazolecarboxylique de formule (I) suivant la revendication 1 ou leurs sels d'addition d'acides ou leurs complexes de sels métalliques sur les micro-organismes indésirables ou les parasites animaux et/ou sur leur milieu.

6. Procédé de préparation de compositions destinées à protéger des plantes d'une attaque par des micro-organismes indésirables et à combattre des parasites animaux, caractérisé en ce qu'on mélange des (thio)esters d'acide 1,2,3-thiadiazolecarboxylique de formule (I) suivant la revendication 1 ou leurs sels d'addition d'acides ou leurs complexes de sels métalliques avec des diluants et/ou des agents tensio-actifs.
